(19) Europäisches Patentamt
European Patent Office
Office européen des brevets

(11) Publication number : **0 070 076 B2**

# (12) NEW EUROPEAN PATENT SPECIFICATION

(45) Date of publication of the new patent specification : **04.11.92 Bulletin 92/45**

(51) Int. Cl.$^5$ : **C11D 1/86,** C11D 1/66, C11D 1/52, C11D 1/75

(21) Application number : **82200870.2**

(22) Date of filing : **12.07.82**

## (54) Foaming dishwashing liquid compositions.

The file contains technical information submitted after the application was filed and not included in this specification

(30) Priority : **26.04.82 US 371695**
**13.07.81 US 282976**

(43) Date of publication of application :
**19.01.83 Bulletin 83/03**

(45) Publication of the grant of the patent :
**21.01.87 Bulletin 87/04**

(45) Mention of the opposition decision :
**04.11.92 Bulletin 92/45**

(84) Designated Contracting States :
**AT BE CH DE FR GB IT LI NL**

(56) References cited :
**FR-A- 2 093 790**
**US-A- 3 721 633**

(56) References cited :
**US-A- 3 839 318**
**US-A- 4 154 706**
**US-A- 4 240 921**
**Fette, Seifen, Anstrichmittel, Die Ernährungsindustrie 68, 1966, pp. 551-556**
**Rohm & Haas, Technical Bulletin, Triton CG-110**

(73) Proprietor : **THE PROCTER & GAMBLE COMPANY**
**301 East Sixth Street**
**Cincinnati Ohio 45202 (US)**

(72) Inventor : **Jones, Keith Anthony**
**5761 Sigmond Way**
**Fairfield, OH 45014 (US)**
Inventor : **Llenado, Ramon Aquillon**
**8090 Pepper Pike**
**West Chester, OH 45069 (US)**

(74) Representative : **Canonici, Jean-Jacques et al**
**Procter & Gamble European Technical Center**
**N.V. Temselaan 100**
**B-1853 Strombeek-Bever (BE)**

EP 0 070 076 B2

## Description

This invention relates to liquid dishwashing detergent compositions which provide controllable aqueous foams.

Alkylpolyglucosides which are surfactants have been disclosed in U.S.Patents 3,598,865; 3,721,633; and 3,772,269. These patents also disclose processes for making alkylpolyglucoside surfactants and built liquid detergent compositions containing these surfactants. U.S.Patent 3,219,656 discloses alkylmonoglucosides and suggests their utility as foam stabilisers for other surfactants. Various polyglucoside surfactant structures and processes for making them are disclosed in U.S.Patents 2,974,134; 3,640,998; 3,839,318; 3,314,558; 4,011,389; 4,223,129. Dishwashing compositions containing anionic surfactants, nonionic surfactants and amine oxides or amides are known from Fette, Seifen, Anstrichmittel, Die Ernähungsindustrie 68, 551-556 (1966).

Foam compositions containing an alkylpolysaccharide surfactant, and anionic cosurfactants are described in copending published EP-A-0070074 and EP-A-0070075.

Light duty detergent compositions containing an alkylpolysaccharide, alkylbenzene sulfonate and alkylpolyethoxylate sulfate cosurfactants are described in copending published EP-A-0070077.

All percentages, parts and ratios used herein are by weight unless otherwise specified.

This invention relates to foaming liquid diswhashing detergents comprising 10-50% (by weight of the composition) of a mixture of surfactants comprising

(1) an alkyl polyglucoside of the formula

$$R^2O(C_nH_{2n}O)_t(Z)_x$$

wherein Z is derived from glucose; $R^2$ is a hydrophobic saturated alkyl group containing from 12 to 18, preferably from 12 to 14 carbon atoms; n is 2 or 3, preferably 2, t is from 0 to 10, preferably 0; and x is from 1.5 to 4, preferably from 1.6 to 2.7, and including less than 10% unreacted fatty alcohol and less than 50% short chain alkyl polyglucoside.

(2) an anionic cosurfactant selected from the group consisting of sulfates, sulfonates, carboxylates and mixtures thereof neutralised with one or more cationic moieties, the weight ratio of (2) to (1) being from 1:10 to 10:1; and

(3) from 2% to 10% (by weight of the composition) of an auxiliary foam booster selected from the group consisting of

(a) amides having the formula

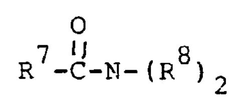

wherein $R^7$ is an alkyl group containing from 8 to 18 carbon atoms and each $R^8$ is the same or different and is selected from the group consisting of hydrogen, $C_{1-3}$ alkyl, $C_{1-3}$ alkanol, and $-(C_2H_4O-)_{1-4}H$ groups and mixtures thereof;

(b) amine oxides having the formula:

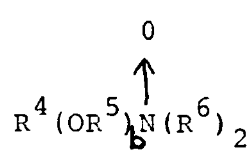

wherein $R^4$ is an alkyl group containing from 8 to 18, preferably from 12 to 14, carbon atoms, each $R^5$ contains two or three carbon atom, b is from 0 to 30, each $R^6$ is the same or different and is selected from the group consisting of $C_{1-3}$ alkyl, $C_{1-3}$ alkanol, and $-(C_2H_4O)_{1-6}H$ groups and mixtures thereof; and

(c) mixtures thereof.

It has surprisingly been found that the cosurfactants interact with the alkylpolyglucoside surfactant to provide a relatively stable foam which is readily rinsed and in particular that gives good suds mileage and "Suds during Washing" values.

The alkylpolyglucoside are those having an alkyl hydrophobic group that is saturated and that contains from 12 to 18 carbon atoms preferably from 12 to 16 carbon atoms. The number x indicates the number of saccharide units in a particular alkylpolyglucoside surfactant. For a particular alkylpolyglucoside molecule x can only assume integral values. In any physical sample of alkylpolysaccharide surfactants there will in general be molecules having different x values. The physical sample can be characterised by the average value of x and this average value can assume non-integral values. In this specification the values of x are to be understood to be average values. The hydrophobic group ($R^2$) can be attached at the 2-, 3-, or 4-positions rather than at the 1-position, (thus giving e.g. a glucosyl as opposed to a glucoside). However, attachment through the 1-position, i.e., glucosides, is preferred. In the preferred product the additional glucoside units are predominately attached to the previous glucoside units's 2-position. Attachment through the 3-, 4-, and 6-positions can also occur.

Optionally and less desirably there can be a polyalkoxide chain joining the hydrophobic moiety ($R^2$) and the polyglucoside chain. The preferred alkoxide moiety is ethoxide.

The alkylmonoglucoside are relatively less soluble in water than the higher alkylpolyglucosides. When used in admixture with alkylpolyglucosides the alkylmonoglucosides are solubilised to some extent. The use of alkylmonoglucosides in admixture with alkylpolyglucosides is a preferred mode of carrying out the invention. Suitable mixtures include coconut alkyl, di-, tri- and, tetraglucosides and tallow alkyl tetraglucosides.

To prepare these compounds a long chain alcohol ($R^2OH$) can be reacted with glucose, in the presence of an acid catalyst to form the desired glucoside. Alternatively the alkylpolyglucosides can be prepared by a two step procedure in which a short chain alcohol ($C_{1-6}$) is reacted with glucose or a polyglucoside (x=2 to 4) to yield a short chain alkyl glucoside (x=1 to 4) which can in turn be reacted with a longer chain alcohol ($R^2OH$) to displace the short chain alcohol and obtain the desired alkylpolyglucoside. If this two step procedure is used, the short chain alkylglucoside content of the final alkylpolyglucoside material should be less than 50%, preferably less than 10%, more preferably less than 5%, most preferably 0% of the alkylpolyglucoside.

The amount of unreacted alcohol (the free fatty alcohol content) in the desired alkylpolyglucoside surfactant is preferably less than 2%, more preferably less than 0.5% by weight of the total of the alkylpolyglucoside plus unreacted alcohol. The amount of alkylmonoglucoside is 20% to 70%, preferably 30% to 60%, most preferably 30% to 50% by weight of the total of the alkylpolyglucoside. For some uses it is desirable to have the alkylmonoglucoside content less than 10%.

Throughout this specification, "alkylglucoside" is used to include alkylglycosides because the sterochemistry of the saccharide moiety is changed during the preparation reaction.

Anionic cosurfactants can be selected from the group consisting of sulfates, sulfonates, carboxylates and mixtures thereof. The cosurfactants are neutralised with a cationic moiety or moieties selected from the group consisting of alkali metal, e.g. sodium or potassium, alkaline earth metal, e.g. calcium or magnesium, ammonium, substituted ammonium, including mono-, di-, or tri-, ethanolammonium cations. Mixtures of cations can be desirable. The anionic cosurfactants useful in the present invention all have detergent properties and are all water-soluble or dispersible in water.

One of the cosurfactants for use in this invention can be an alkylbenzene sulfonate. The alkyl group can be either saturated or unsaturated, branched or straight chain and is optionally substituted with a hydroxy group. Middle phenyl positions are generally preferred for volume of foaming in light soil conditions. However, in heavier soil conditions, phenyl attachment at the 1- or 2-position is preferred.

The preferred alkylbenzene sulfonates contain a straight alkyl chain containing from 9 to 25 carbon atoms, preferably from 10 to 13 carbon atoms, and the cation is sodium, potassium, ammonium, mono-, di-, or triethanolammonium, calcium or magnesium and mixtures thereof. Magnesium is the preferred cationic moiety. These same cations are preferred for other anionic surfactants and ingredients. The magnesium alkylbenzene sulfonates where the phenyl group is attached near the middle of the alkyl chain are surprisingly better than the ones with the phenyl near the end of the chain when the polyglucoside chain averages greater than 3 glucosides units. Suitable alkylbenzene sulfonates include $C_{11}$ alkylbenzene sulfonates with low 2-phenyl content.

Many other surfactants which contain sulfonate or carboxylate groups can be used in the foaming compositions of the invention. Generally the use of these latter cosurfactants produces less foam volume than does the use of the preferred cosurfactants. However, the alkylpolysaccharide surfactant stabilises the foams which are produced and allows the foams to be rinsed more quickly.

One group of cosurfactants that are of interest because of their superior detergency are the zwitterionic detergents which contain both a cationic group, either ammoniun, phosphonium, sulfonium or mixtures thereof and a sulfonate or carboxylate group. Preferably there are at least about four atoms separating the cationic and anionic groups. Suitable zwitterionic surfactants are disclosed in U.S.Patents 4,159,277; 3,928,251; 3,925,262; 3,929,678; 3,227,749; 3,539,521; 3,383,321; 3,390,094; and 3,239,560.

Another group of cosurfactants are the amphoteric detergents which have the same general structure as the zwitterionic surfactants but with an amine group instead of the quaternary ammonium group.

Yet other cosurfactants are the alkyl (paraffin or olefin) sulfonates, preferably with a more central hydrophilic group, containing from 6 to 30 carbon atoms. Compositions containing these cosurfactants produce the

least volume of foam., if that is desired. The hydrophobic group can contain up to 10 hydroxy groups and/or ether linkages. Examples include $C_{14-15}$ paraffin sulfonates and $C_{14-16}$ olefin sulfonates.

Still another cosurfactant is a soap structure containing up to 10 ether linkages in the chain and from 1 to 4 carbon atoms between ether linkages with from 6 to 30 carbon atoms in a terminal portion containing no ether linkages.

The alkylpolyglucosides can be contaminated with less than 50% short chain alkylpolyglucosides and with less than 10%, preferably less than 2%, most preferably less than 0.5% unreacted fatty alcohol and increase the sudsing ability of conventional sulfate detergent cosurfactants, especially alkyl sulfate and alkyl polyether sulfate cosurfactants having the formula:

$$R^3O(C_nH_{2n}O)_tSO_3M$$

wherein $R^3$ is an alkyl or hydroxyalkyl group containing from 8 to 18 carbon atoms, n is 2 or 3, t can vary from 0 to 30, and M is a cationic moiety as defined above, the cosurfactant being water soluble or dispersible.

The compositions of the invention provide superior suds mileage.

Preferred anionic cosurfactants are alkylbenzene sulfonate, alpha-olefin sulfonate, alkylsulfates, alkylpolyethoxylate sulfates an paraffin sulfonates and mixtures thereof. The cationic moieties are selected from the group consisting of sodium, potassium, ammonium, monoethanolammonium, diethanolammonium, triethanolammonium, triethanolammonium, calcium, magnesium and mixtures thereof.

Preferred compositions of this embodiment of the invention comprise from 1% to 95%, preferably 5% to 50% of an akylpolyglucoside surfactant in which the alkyl group contains from 12 to 14 carbon atoms, x is from 1.5 to 4, more preferably 1.5 to 2.7; from 1% to 95%, preferably from 10% to about 50% of an anionic cosurfactant neutralised with one or more cationic moieties and which is a mixture of

(1) from 1% to 95%, preferably from 5% to 50% of an alkyl benzene sulfonate in which the alkyl group contains from 8 to 13 carbon atoms or an alpha-olefin sulfonate in which the olefin group contains from 10 to 18 carbon atoms, or mixtures thereof; and

(2) from 1% to 95%, preferably from 5% to 50% of an alkyl polyethoxylate sulfate in which the alkyl group contains from 8 to 18 carbon atoms, preferably from 12 to 14 carbon atoms and from one to six ethoxylate moieties and wherein from 1% to 100%, preferably from 10% to 80% of the cationic moieties are magnesium; and wherein the auxiliary foam booster is an amide.

Typical compositions for use as light duty liquid detergent compositions in washing dishes comprise from 10% to 50%, preferably from 10% to 40% of the mixture of surfactants disclosed hereinbefore and from 1% to 50% of a solvent selected from the group consisting of $C_{1-3}$ alkanols, $C_{1-3}$ alkanolamines, $C_{2-4}$ polyols, mixtures thereof, and the balance water. It is a special advantage of the compositions of this invention that they can be made in concentrated form (up to 50% by wt. of the mixture of surfactants) with only very low levels of organic solvents and without the addition of expensive hydrotropic materials. Fatty alcohols should not be used.

The compositions of this invention can utilise other compatible ingredients, including other surfactants, in addition to the mixture of cosurfactants herein disclosed.

The following nonlimiting examples illustrate the foaming compositions of the present invention.

The relative volume of suds in ml is determined by the following test procedure:

100ml of the test solution at 46.1°C is placed in a 500ml graduated cylinder: the solution is agitated by repeated inversion of the graduated cylinder until the amount of suds in the cylinder does not increase with further agitation. Suds height is measured directly on the cylinder scale making allowance for the height of liquid remaining in the cylinder. The test solution is made by adding the test product to water having a hardness of 0.12 grams per liter (Ca/Mg=3/1).

Example 1

The following formulas were prepared:

| | A | B | C |
|---|---|---|---|
| Magnesium linear $C_{11.2}$ alkylbenzene sulfonate | 22.4 | 22.4 | 22.4 |
| $C_{12-13}$ alkylpolyglucoside ($G_{1.7}$) (<2% free fatty alcohol) | 14.9 | 14.9 | 14.9 |
| $C_{9-11}$ alkoxypropyldihydroxyethyl amine oxide | _ | 4 | _ |
| $C_{12}$ alkyldihydroxy ethyl amine oxide | _ | _ | 4 |
| Ethanol | 5 | 5 | 5 |
| Water | balance | balance | balance |

Formulas A, B and C were compared by generating suds with a constant source of agitation under standard conditions [3.785 l (1 gal) water, 46.1°C (115°F) 0.12 g/l hardness in a 11.3 dishpan using a standardised mixture of fat plus protein, carbohydrate and edible acid]. Dinner plates are washed with 4ml of soil on each plate and the suds height is measured after each five plates. 30 plates in total are washed and the integral of the suds height taken over the number of plates washed is reported as the SDW grade (SDW = Suds During Washing).

| | A | B | C |
|---|---|---|---|
| SDW grade | 24 | 28.8 | 28.4 |

This shows that the addition of a small amount of these amine oxides dramatically increases the amount of dishes that can be washed. Similar results are obtained when a fatty acid amide, e.g., a coconut fatty acid amide, diethanol amide, and/or isopropanol amide is substituted, at least in part for the specific amide oxides.

<u>Exemple 2</u>

The following formulas were prepared:

| | % by weight | | | |
|---|---|---|---|---|
| | A | B | C | D |
| Ammonium/magnesium $C_{11.2}$ linear alkyl benzene sulfonate | 24.2 | 21.8 | _ | _ |
| Ammonium/magnesium $C_{12-15}$ olefin sulfonate | _ | _ | 12.8 | 10.6 |
| Ammonium/magnesium $C_{12-13}$ alkyl sulfate | _ | _ | 19.2 | 15.9 |
| Ammonium $C_{12-13}$ alkyl polyethoxylate (0.8) sulfate | 6.5 | 5.8 | _ | _ |
| $C_{12}$ fatty acid diethanolamide | _ | 3.8 | _ | 5.5 |
| $C_{12-13}$ alkylpoly-glucoside $G_{1.7}$ (free fatty alcohol <0.5%) | 5.3 | 4.8 | 4 | 3.3 |
| Minors and water | | balance | | |

| | A | B | C | D |
|---|---|---|---|---|
| The SDW index | 79 | 89 | 97 | 107 |

The SDW index is the SDW grade for each product as a percentage of the SDW value of a standard commercial product.

The following is an example of particularly preferred compositions. The broad and preferred ranges of ingredients which can be used are given in the second and third columns, respectively, in each example.

Example 3

| | % by weight | | |
|---|---|---|---|
| Ammonium $C_{12-13}$ alkyl sulfate | 15.7 | 7-23 | 10-20 |
| Sodium $C_{14-16}$ olefin sulfonate | 10.4 | 4-19 | 6-13 |
| $MgCI_2$ $6H_2O$ | 5.6 | 0-11 | 2-10 |
| Coconut monoethanol amide | 5.5 | 2-8 | 3-7 |
| $C_{12-13}$ alkyl polyglucoside (1.7) derived from glucose (<0.5% free fatty alcohol) | 5.9 | 2-12 | 3-9 |
| Ethanol | 4.0 | 0-10 | 0-10 |
| $H_2O$ and minor components, e.g., perfume | | balance | |

Known analytical techniques can be used to determine the structures of the alkylpolyglucoside surfactants herein; for example to determine the glycosidic chain length, the amount of butyl glucoside, the free fatty alcohol content, and the level of unreacted polyglucoside. More specifically, gas or liquid chromatrography can be used to determine the unreacted alcohol content and the unreacted polyglucoside content respectively. Proton nmr can be used to determine the average glycosidic chain length. The point of attachment of the hydrophilic portion of the molecule to the hydrophobic portion of the molecule can be determined by $^{13}C$ nmr.

The alkylpolyglucoside surfactants are complex mixtures. Their components vary depending upon the nature of the starting materials and the reaction by which they are prepared. Analytical standards which are useful in calibrating instruments for analysing the components of a particular alkylpolyglucoside surfactant can be obtained from Calbiochem Behring Co.Lajolla, California. These standards include those for octylglucoside (Calbiochem #494559), decylglucoside (Calbiochem #252715), dodecylmaltoside (Calbiochem #3243555).

## Claims

1. A foaming dishwashing liquid detergent composition comprising 10-50% (by weight of the composition) of a mixture of surfactants comprising
   (1) an anionic cosurfactant selected from the group consisting of sulfates, sulfonates, carboxylates and mixtures thereof neutralised with one or more cationic moieties, and
   (2) from 2% to 10% (by weight of the composition) of an auxiliary foam booster selected from the group

consisting of:

(a) amides having the formula

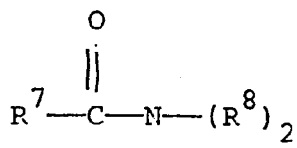

wherein $R^7$ is an alkyl group containing from 8 to 18 carbon atoms and each $R^8$ is the same or different and is selected from the group consisting of hydrogen, $C_{1-3}$ alkyl, $C_{1-3}$ hydrokyalkyl (and -$(C_2H_4O\text{-})_{1-4}H$ groups and mixtures thereof;

(b) amine oxide having the formula

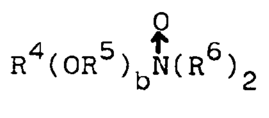 ,

wherein $R^4$ is an alkyl group containing from 8 to 18 carbon atoms, each $R^5$ is ethylene or propylene, b is from 0 to 30, each $R^6$ is the same or different and is selected from the group consisting of $C_{1-3}$ alkyl, $C_{1-3}$ hydrokyalkyl and -$(C_2H_4O)_{1-6}H$ groups and mixtures thereof; and

(c) mixtures thereof,

characterised in that the mixture also includes

(3) an alkylpolyglucoside surfactant having the formula

$$R^2O(C_nH_{2n}O)_tZ_x$$

wherein Z is a moiety derived from glucose; $R^2$ is an alkyl group that contains from 12 to 18 carbon atoms; t is from 0 to 10; and x is from 1.5 to 4, n is 2 or 3, and including less than 10% unreacted fatty alcohol and less than 50% short chain alkyl polyglucosides, the weight ratio of (1) : (3) being from 1:10 to 10:1.

**2.** A composition according to claim 1 in which x is 1.6 to 2.7.

**3.** A composition according to any preceding claim wherein the cosurfactant is selected from the group consisting of alkylbenzene sulfonates, alpha-olefin sulfonates, alkyl sulfates, alkyl polyethoxylate sulfates, and paraffin sulfonates and mixtures thereof and the cationic moiety is selected from the group consisting of sodium, potassium, ammonium, monoethanolammonium, diethanolammonium, triethanolammonium, calcium, magnesium and mixtures thereof.

**4.** The composition of claim 3 wherein the cosurfactant is an alkylbenzene sulfonate.

**5.** The composition of claim 4 wherein the phenyl portion of the alkylbenzene sulfonate is attached near the middle of the alkyl chain and at least 10% of the cations are magnesium.

**6.** The composition of claim 3 wherein the cosurfactant is a mixture of

(1) from 5% to 50% by weight of an alkyl benzene sulfonate in which the alkyl group contains from 8 to 13 carbon atoms or an alpha-olefin sulfonate in which the olefin group contains from 10 to 18 carbon atoms, or mixtures thereof, and

(2) from 5% to 50% by weight of an alkyl polyethoxylate sulfate in which the alkyl group contains from 10 to 18 carbon atoms and from one to six ethoxylate moieties and from 10% to 80% by weight of the cationic moieties are magnesium.

## Patentansprüche

**1.** Schäumende, flüssige Geschirrspüldetergenszusammensetzung, enthaltend 10 % bis 50 % (bezogen auf das Gewicht der Zusammensetzung) von einem Gemisch grenzflächenaktiver Mittel, enthaltend

(1) ein anionisches, co-grenzflächenaktives Mittel, ausgewählt aus der Gruppe, die aus mit einem oder mehreren kationischen Resten neutralisierten Sulfaten, Sulfonaten, Carboxylaten, und Mischungen davon, besteht; und

(2) 2 % bis 10 % (bezogen auf das Gewicht der Zusammensetzung) eines Hilfsschaumverstärkers, ausgewählt aus der Gruppe, die aus:

(a) Amiden der Formel

$$R^7 - \overset{O}{\overset{\|}{C}} - N - (R^8)_2 ,$$

worin $R^7$ eine 8 bis 18 Kohlenstoffatome enthaltende Alkylgruppe ist, und jeder Rest $R^8$ gleich oder vom anderen verschieden ist und aus der Gruppe ausgewählt ist, die aus Wasserstoff, $C_{1-3}$-Alkyl-, $C_{1-3}$-Hydroxyalkyl- und $-(C_2H_4O-)_{1-4}H$-Gruppen, und Mischungen davon, besteht;

(b) Aminoxiden der Formel

$$R^4(OR^5)_b\overset{O}{\overset{\uparrow}{N}}(R^6)_2 ,$$

worin $R^4$ eine 8 bis 18 Kohlenstoffatome enthaltende Alkylgruppe ist, jeder Rest $R^5$ Ethylen oder Propylen ist, b 0 bis 30 ist, jeder Rest $R^6$ gleich oder vom anderen verschieden ist und aus der Gruppe ausgewählt ist, die aus $C_{1-3}$-Alkyl-, $C_{1-3}$-Hydroxyalkyl- und $-(C_2H_4O)_{1-6}H$-Gruppen, und Mischungen davon, besteht; und

(c) Mischungen davon, besteht,

dadurch gekennzeichnet, daß das Gemisch auch ein

(3) Alkylpolyglucosid-grenzflächenaktives Mittel der Formel

$$R^2O(C_nH_{2n}O)_tZ_x$$

enthält, worin Z ein sich aus Glusoce herleitender Rest ist; $R^2$ eine Alkylgruppe darstellt, welche 12 bis 18 Kohlenstoffatome enthält; t 0 bis 10 beträgt; und x 1,5 bis 4 ist, n 2 oder 3 ist, und welches weniger als 10 % an nicht-umgesetztem Fettalkohol und weniger als 50 % an kurzkettigen Alkylpolyglucosiden aufweist, wobei das Gewichtsverhältnis von (1):(3) von 1:10 bis 10:1 beträgt.

2. Eine Zusammensetzung nach Anspruch 1, wobei x von 1,6 bis 2,7 ist.

3. Eine Zusammensetzung nach einem der vorstehenden Ansprüche, wobei das co-grenzflächenaktive Mittel aus der Gruppe ausgewählt ist, die aus Alkylbenzolsulfonaten, $\alpha$-Olefinsulfonaten, Alkylsulfaten, Alkylpolyethoxylatsulfaten und Paraffinsulfonaten, und Mischungen davon, besteht, und der kationische Rest aus der Gruppe ausgewählt ist, die aus Natrium, Kalium, Ammonium, Monoethanolammonium, Diethanolammonium, Triethanolammonium, Calcium, Magnesium, und Mischungen davon, besteht.

4. Die Zusammensetzung des Anspruchs 3, wobei das co-grenzflächenaktive Mittel ein Alkylbenzolsulfonat ist.

5. Die Zusammensetzung des Anspruchs 4, wobei der Phenylabschnitt des Alkylbenzolsulfonats nahe der Mitte der Alkylkette gebunden ist, und wenigstens 10 % der Kationen Magnesium sind.

6. Die Zusammensetzung des Anspruchs 3, wobei das co-grenzflächenaktive Mittel ein Gemisch aus

(1) 5 Gew.-% bis 50 Gew.-% eines Aklylbenzolsulfonats, in welchem die Alkylgruppe 8 bis 13 Kohlenstoffatome enthält, oder eines $\alpha$-Olefin-sulfonats, in welchem die Olefingruppe 10 bis 18 Kohlenstoffatome enthält, oder von Mischungen davon; und

(2) 5 Gew.-% bis 50 Gew.-% eines Alkylpolyethoxylatsulfats, in welchen die Alkygruppe 10 bis 18 Kohlenstoffatome und 1 bis 6 Ethoxylatreste enthält, und 10 Gew.-% bis 80 Gew.-% der kationischen Reste Magnesium sind, ist.

## Revendications

1. Composition détergente liquide moussante pour le lavage de la vaisselle comprenant 10 à 50 % (en poids de la composition) d'un mélange d'agents tensio-actifs comprenant :

(1) un co-agent tensio-actif anionique choisi parmi le groupe constitué des sulfates, sulfonates, carboxylates et de leurs mélanges neutralisés par un ou plusieurs groupes cationiques,

(2) 2 % à 10 % (en poids de la composition) d'un amplificateur de mousse auxiliaire choisi dans le groupe constitué de :

(a) d'amides ayant pour formule :

$$R^7-\overset{O}{C}-N-(R^8)_2$$

dans laquelle $R^7$ est un groupe alkyle contenant 8 à 18 atomes de carbone et chaque groupe $R^8$ est identique au différent et est choisi parmi le groupe constitué de l'hydrogène, des groupes alkyle en $C_{1-3}$, hydroxyalkyle en $C_{1-3}$ et $-(C_2H_4O-)_{1-4}H$ et de leurs mélanges,

(b) d'oxydes d'amines ayant pour formule :

$$R^4(OR^5)_b\overset{O}{N}(R^6)_2$$

dans laquelle $R^4$ est un groupe alkyle contenant 8 à 18 atomes de carbone, chaque groupe $R^5$ est de l'éthylène ou du prolypène, b est un nombre de de 0 à 30, chaque groupe $R^6$ est identique au différent et est choisi parmi le groupe constitué des groupes alkyle en $C_{1-3}$, hydroxyalkyle en $C_{1-3}$ et $-(C_2H_4O)_{1-6}H$ et de leurs mélanges; et

(c) de leurs mélanges,

caractérisée en ce que le mélange comprend également :

(3) un agent tensio-actif de type alkylpolyglucoside ayant pour formule

$$R^2O(C_nH_{2n}O)_tZ_x$$

dans laquelle Z est un groupe dérivé du glucose; $R^2$ est un groupe alkyle qui contient 12 à 18 atomes de carbone; t est un nombre de 0 à 10; et x est un nombre de 1,5 à 4, n est égal à 2 ou 3, et comprenant moins de 10 % d'alcool gras qui n'a pas réagi et moins de 50 % d'alkylpolyglucosides à chaîne courte, le rapport pondéral de (1) : (3) étant de 1 : 10 à 10 : 1.

2. Composition selon la revendication 1, dans laquelle x est un nombre de 1,6 à 2,7.

3. Composition selon l'une quelconque des revendications précédentes, dans laquelle le co-agent tensio-actif est choisi parmi le groupe constitué des alkylbenzène sulfonates, des alpha oléfine sulfonates, des alkyl sulfates, des alkyl polyéthoxylate sulfates et des paraffine sulfonates et de leurs mélanges et le groupe cationique est choisi parmi le groupe constitué du sodium, du potassium, de l'ammonium, du monoéthanolammonium, du diéthanolammonium, du triéthanolammonium, du calcium, du magnésium et de leurs mélanges.

4. Composition selon la revendication 3, dans laquelle le co-agent tensio-actif est un alkyl benzène sulfonate.

5. Composition selon la revendication 4, dans laquelle la partie phényle de l'alkyl benzène sulfonate est fixée à proximité du centre de la chaîne alkyle et au moins 10 % des cations sont constitués de magnésium.

6. Composition selon la revendication 3, dans laquelle le co-agent tensio-actif est un mélange de :

(1) 5 % à 50 % en poids d'un alkyl benzène sulfonate dans lequel le groupe alkyle contient 8 à 13 atomes de carbone ou d'un alpha oléfine sulfonate dans lequel le groupe oléfine contient 10 à 18 atomes de carbone, ou de leurs mélanges, et

(2) 5 % à 50 % en poids d'un alkyl polyéthoxylate sulfate dans lequel le groupe alkyle contient 10 à 18 atomes de carbone et et un à six groupes éthoxylate et 10 % à 80 % en poids des groupes cationiques sont constitués de magnésium.